Europäisches Patentamt

(19) European Patent Office

Office européen des brevets

(11) Numéro de publication : **0 314 578 B1**

(12) **FASCICULE DE BREVET EUROPEEN**

(45) Date de publication du fascicule du brevet :
29.05.91 Bulletin 91/22

(51) Int. Cl.⁵ : **A61F 5/455**

(21) Numéro de dépôt : **88402723.6**

(22) Date de dépôt : **28.10.88**

(54) **Réceptacle collecteur permanent d'urine pour femme.**

(30) Priorité : 30.10.87 FR 8715093

(43) Date de publication de la demande :
03.05.89 Bulletin 89/18

(45) Mention de la délivrance du brevet :
29.05.91 Bulletin 91/22

(84) Etats contractants désignés :
ES GR

(56) Documents cités :
EP-A- 0 185 517
US-A- 4 583 983

(73) Titulaire : KILRUSH LIMITED
49 Conduit Street
London W1R 9FB (GB)

(72) Inventeur : Nigay, Pierre
10 rue de Paris
F-60200 Compiegne (FR)

(74) Mandataire : Bertrand, Didier et al
c/o S.A. Fedit-Loriot 38, avenue Hoche
F-75008 Paris (FR)

Jouve, 18, rue Saint-Denis, 75001 PARIS

fond de la rigole. Les orifices 11 servent d'évents lors du remplissage de l'enceinte 8 par la cheminée 5.

Vers la partie avant supérieure de la zone intérieure à la lèvre 4, une cuvette allongée 12 peut recueillir le liquide et le diriger vers la rigole 10.

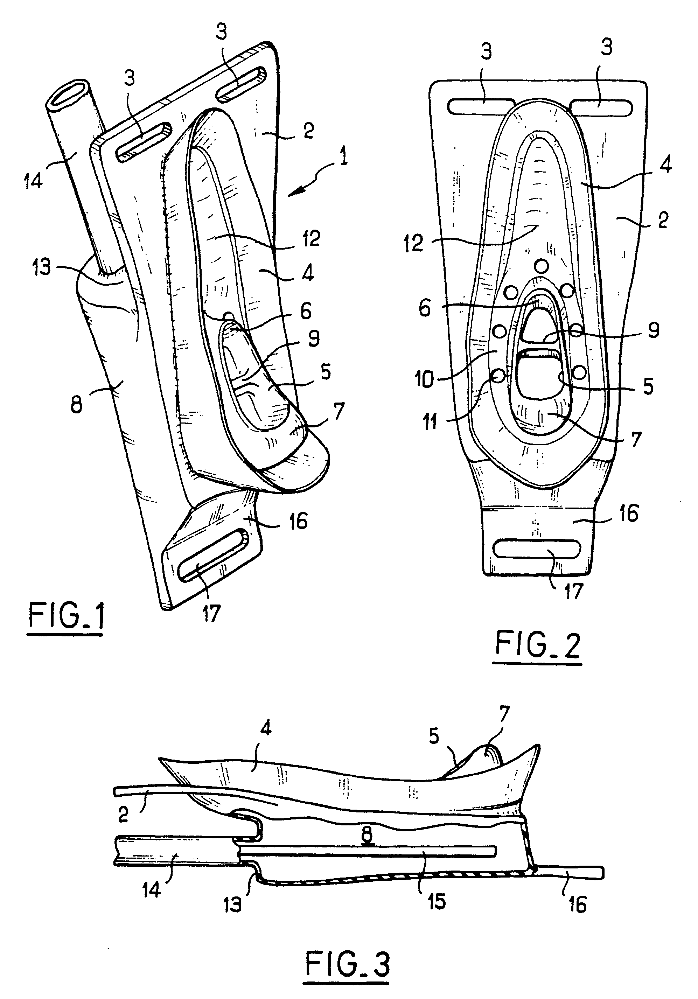

En dessous du plateau 2, l'enceinte 8 est pourvue, sur une paroi frontale avant supérieure 13, d'un tube d'évacuation 14 relié à l'ensemble de pompage décrit dans les brevets précités. On a représenté sur la figure 3, l'extrémité du tuyau d'aspiration 15.

La forme de l'enceinte 8, et la position du tuyau d'aspiration 15 et des électrodes de détection sont avantageusement prévues pour conserver dans la poche une petite quantité permanente de liquide qui évite des phénomènes de bruit liés à la présence d'air dans les circuits de pompage.

Au bas de l'enceinte 8 est prévue une patte 16 pourvue d'une fente 17 par le passage d'une sangle inférieure de fixation. Le cas échéant, cette patte de support 16 peut être reliée à un dispositif de rétention des selles qui fait suite au dispositif collecteur d'urine.

## Revendications

1. Réceptacle collecteur permanent pour recueillir l'urine d'une femme, du type comportant un plateau et une cheminée oblongue biseautée destinée à coopérer avec l'urètre, caractérisé en ce que le plateau comporte une lèvre relevée souple (4) conformée pour entourer les organes féminins, dans la zone intérieure de laquelle se trouve ladite cheminée (5), entourée d'une rigole (10), et, d'un côté seulement, d'une cuvette (12), la cheminée (5) et la rigole (10) communiquant avec une enceinte collectrice (8) attenante au plateau.

2. Réceptacle selon la revendication 1, caractérisé en ce que l'enceinte collectrice (8) comporte des moyens d'évacuation (14, 15).

3. Réceptacle selon la revendication 2, caractérisé en ce que les moyens d'évacuation sont associés à un système de pompage.

## Claims

1. A permanent urine-collecting receptacle for women, of the type having a plate and a beveled oblong duct for cooperating with the urethra, characterized in that the plate has a flexible raised lip (4) shaped so as to surround the female organs, the said duct (5) being located in the interior zone of the said lip and being surrounded by a channel (10) and, on one side only, by a dish (12), and the duct (5) and the channel (10) communicating with a collecting chamber (8) adjoining the plate.

2. The receptacle according to claim 1, characterized in that collecting chamber (8) has discharge means (14, 15).

3. The receptacle according to claim 2, characterized in that the discharge means are associated with a pumping system.

## Ansprüche

1. Permanenter Sammelbehälter zum Auffangen des Urins einer Frau, der Art, die über eine Platte und einen länglichen, abgeschrägten Kaminabzug verfügt, der zur Zusammenarbeit mit der Harnröhre bestimmt ist, dadurch gekennzeichnet, daß die Platte über eine hochgezogene weiche Lippe (4) verfügt, die so gestaltet ist, daß sie die weiblichen Organe umgibt, in deren inneren Zone sich der Kaminabzug (5) befindet, der von einem Abzugkanal (10) und auf lediglich einer Seite von einer Küvette (12) umgeben ist, wobei der Kaminabzug (5) und der Abzugkanal (10) mit einem an die Platte anstoßenden Sammelraum (8) in Verbindung stehen.

2. Behälter nach Anspruch 1, dadurch gekennzeichnet, daß der Sammelraum (8) über Evakuierungsmittel (14, 15) verfügt.

3. Behälter nach Anspruch 2, dadurch gekennzeichnet, daß die Evakuierungsmittel an ein Pumpensystem angeschlossen sind.

FIG.1

FIG.2

FIG.3